# EUROPEAN PATENT APPLICATION

(11) **EP 1 604 671 A1**
(43) Date of publication of application: **14.12.2005**
(21) Application number: 04714920.8
(22) Date of filing: 26.02.2004
(51) Int. Cl.: A61K 31/7068, A61P 21/00, A61P 43/00, A23K 1/16, A23K 1/18, A23L 1/30

(54) **MUSCLE-BUILDING AGENT AND PREVENTIVE OR REMEDY FOR MUSCLE WEAKENING**

(30) Priority: 26.02.2003 JP 2003048589
(71) Applicant: KYOWA HAKKO KOGYO CO., LTD., Chiyoda-ku, Tokyo 100-8185 (JP)
(72) Inventor: KOMATSU, Miho,c/o Healthcare Research Laboratories, Tsukuba-shi, Ibaraki 305-0841 (JP); KAMIYA, Toshikazu, c/o Healthcare Research Laborat, Tsukuba-shi, Ibaraki 305-0841 (JP)
(74) Representative: Harding, Charles Thomas
(86) International application number: PCT/JP2004/002269
(87) International publication number: WO 2004/075905

(57) **Abstract**

In accordance with the present invention, there can be provided a muscle building agent, a preventive or therapeutic agent for muscle wasting, food and drink or feed for muscle building or for prevention or treatment of muscle wasting, and a food and drink additive or a feed additive for muscle building or for prevention or treatment of muscle wasting which contain cytidine diphosphate choline or a salt thereof.

## Description

### Technical Field

The present invention relates to a muscle building agent and a preventive or therapeutic agent for muscle wasting. The present invention also relates to food and drink, feed, food and drink additive or feed additive for building muscle or for prevention or treatment of muscle wasting.

### Background Art

Athletes wishing to improve their athletic ability undergo training and diet programs for building muscle. Muscle building is expected to be effective not only for improvement in athletic ability but also for activation of basal metabolism and promotion of burning of fat. With regard to a muscle building agent, the effect of growth hormone (hereinafter abbreviated as GH) has been investigated [Sports Medicine, 24, 366 (1997)], but growth hormone has problems in that it is used in doping and has side effects because it acts also on tissues other than muscle. In diet, ingestion of proteins and amino acids which are materials for muscle is recommended.

In the case of orthopedic diseases or of accidents and diseases, muscular atrophy is caused by inactiveness during a rest cure. It is known that antigravity muscle and other muscles become atrophied and become smaller in a weightless state such as in space.

In addition to the above cases, geriatric muscular atrophy as a result of aging is observed in aged people and, since in such cases the person is likely to become bedridden, strengthening of muscle is often much desired. A typical method for the prevention of muscle wasting is appropriate daily physical exercise. However, physical exercise is limited during the rest cure stage, and such an exercise is a big burden for aged, less active people. With regard to enlargement of muscle which has been known as a physiological adaptation phenomenon accompanying physical exercise, its physiological and chemical mechanism has not been clarified yet. Further, no therapeutic agent for muscle wasting has been established yet.

There is a demand for the development of a muscle building agent or a drug for effective prevention or treatment of muscle wasting or for the development of food and drink, food and drink additive, animal feed or animal feed additive capable of building muscle or effectively preventing or treating muscle wasting.

Cytidine diphosphate choline (hereinafter abbreviated as CDP-choline) is a substrate for acetylcholine, which is a neurotransmitter, or for phosphatidylcholine, which is a constituent of cell membrane, *in vivo* [J. Biol. Chem., 222, 193 (1956)]. CDP-choline suppresses the formation of free fatty acids such as arachidonic acid when it is incorporated in phosphatidylcholine of cell membrane [Neurochemical Research, 6, 821 (1981)], and therefore, disorders caused by formation of free fatty acids from phosphatidylcholine which occur, for example, during ischemia can be suppressed by ingestion of CDP-choline. Because of such action, CDP-choline has been used as an activating agent for brain metabolism, and its effect of improving the prognosis of cerebral apoplexy and pharmacological effects on Alzheimer's disease, Parkinson's disease, glaucoma, etc. have been reported [Methods & Findings in Experimental & Clinical Pharmacology, 17, 1 (1995) and Ophthalmology, 106, 1126 (1999)]. However, up to now, no action of CDP-choline on muscle has been reported at all.

### Disclosure of the Invention

An object of the present invention is to provide a muscle building agent or a preventive or therapeutic agent for muscle wasting. Another object of the present invention is to provide food and drink, feed, food and drink additive or feed additive for building muscle or for prevention or treatment of muscle wasting.

The present invention relates to the following (1) to (40).
(1) A muscle building agent comprising CDP-choline or a salt thereof.
(2) The muscle building agent according to the above (1), wherein the muscle is skeletal muscle.
(3) A food and drink for building muscle comprising CDP-choline or a salt thereof.
(4) A food and drink for building muscle comprising CDP-choline or a salt thereof added thereto.
(5) The food and drink for building muscle according to the above (3) or (4), wherein the muscle is skeletal muscle.
(6) A feed for building muscle comprising CDP-choline or a salt thereof.
(7) A feed for building muscle comprising CDP-choline or a salt thereof added thereto.
(8) The feed for building muscle according to the above (6) or (7), wherein the muscle is skeletal muscle.
(9) A food additive for building muscle comprising CDP-choline or a salt thereof.
(10) A food additive for building muscle comprising CDP-choline or a salt thereof added thereto.
(11) The food and drink additive for building muscle according to the above (9) or (10), wherein the muscle is skeletal muscle.
(12) A feed additive for building muscle comprising CDP-choline or a salt thereof.
(13) A feed additive for building muscle comprising CDP-choline or a salt thereof added thereto.
(14) The food additive for building muscle according to the above (12) or (13), wherein the muscle is skeletal muscle.
(15) A method for building muscle, which comprises administering to an animal an effective amount of
   CDP-choline or a salt thereof.
(16) The method according to the above (15), wherein the animal is an animal other than a human being.
(17) The method according to the above (16), wherein the animal other than a human being is livestock, poultry or farm-raised fish.
(18) A preventive or therapeutic agent for muscle wasting comprising CDP-choline or a salt thereof.
(19) The preventive or therapeutic agent for muscle wasting according to the above (18), wherein the muscle is skeletal muscle.
(20) A food and drink for the prevention or treatment of muscle wasting comprising CDP-choline or a salt thereof.
(21) A food and drink for the prevention or treatment of muscle wasting comprising CDP-choline or a salt thereof added thereto.
(22) The food and drink for the prevention or treatment of muscle wasting according to the above (20) or (21), wherein the muscle is skeletal muscle.
(23) A feed for the prevention or treatment of muscle wasting comprising CDP-choline or a salt thereof.
(24) A feed for the prevention or treatment of muscle wasting comprising CDP-choline or a salt thereof added thereto.
(25) The feed for the prevention or treatment of muscle wasting according to the above (23) or (24), wherein the muscle is skeletal muscle.
(26) A food additive for the prevention or treatment of muscle wasting comprising CDP-choline or a salt thereof.
(27) A food additive for the prevention or treatment of muscle wasting comprising CDP-choline or a salt thereof added thereto.
(28) The food additive for the prevention or treatment of muscle wasting according to the above (26) or (27), wherein the muscle is skeletal muscle.
(29) A feed additive for the prevention or treatment of muscle wasting comprising CDP-choline or a salt thereof.
(30) A feed additive for the prevention or treatment of muscle wasting comprising CDP-choline or a salt thereof added thereto.
(31) The food additive for the prevention or treatment of muscle wasting according to the above (29) or (30), wherein the muscle is skeletal muscle.
(32) A method for preventing or treating muscle wasting, which comprises administering to an animal an effective amount of CDP-choline or a salt thereof.
(33) The method according to the above (32), wherein the animal is an animal other than a human being.
(34) The method according to the above (33), wherein the animal other than a human being is livestock, poultry or farm-raised fish.
(35) Use of cytidine diphosphate choline or a salt thereof for the manufacture of a muscle building agent or food and drink, feed, food additive or feed additive for building muscle.
(36) Use of CDP-choline or a salt thereof for the manufacture of a preventive or therapeutic agent for muscle wasting or of food and drink, feed, food additive or feed additive for the prevention or treatment of muscle wasting.

The muscle building agent or preventive or therapeutic agent for muscle wasting comprising CDP-choline or a salt thereof in accordance with the present invention includes a muscle building agent or a preventive or therapeutic agent for muscle wasting comprising either CDP-choline or a salt thereof, a muscle building agent or a preventive or therapeutic agent for muscle wasting comprising both CDP-choline and a salt thereof together, and a muscle building agent or a preventive or therapeutic agent for muscle wasting comprising one or more kinds of CDP-choline salts together.

CDP-choline can be obtained by methods such as chemical synthesis and fermentation. CDP-choline may also be obtained by purchasing a commercially available product.

Chemical synthesis of CDP-choline can be carried out by the method described in J. Biol. Chem., 222, 185 (1956) or by a combination of known chemical synthesis techniques.

To prepare CDP-choline by fermentation, the method described in Biosci. Biotech. Biochem., 61, 956 (1997) may be used.

It is also possible to purchase CDP-choline from Sigma-Aldrich or other companies.

If necessary, the muscle building agent and the preventive or therapeutic agent for muscle wasting comprising CDP-choline or a salt thereof according to the present invention may comprise one or more kinds of pharmaceutically acceptable carriers and, if still necessary, another effective ingredient for the treatment.

The muscle building agent and the preventive or therapeutic agent for muscle wasting according to the present invention can be produced by mixing CDP-choline or a salt thereof with a carrier, as may be required, employing a method which is well known in the technical field of pharmaceuticals.

The pharmaceutical preparations of the present invention include oral and parenteral preparations, preferably, oral preparations and parenteral preparations which can be intravenously, intraperitoneally or subcutaneously administered.

In making the pharmaceutical preparation of the present invention in the form of an oral preparation, it is possible to use additives such as an excipient, a binder, a disintegrating agent, a lubricant, a dispersing agent, a suspending agent, an emulsifier, a diluting agent, a buffer, an antioxidant and an antibacterial agent.

Examples of the dosage forms of the oral preparations are tablets, powders, granules, emulsion, syrup and capsules. When the dosage form of the oral preparation is tablets, powders, granules, etc., the preparation can be prepared by adding excipients such as sugars (e.g., lactose, white sugar, glucose, sucrose, mannitol and sorbitol), starch (e.g., potato starch, wheat starch and corn starch), inorganic substances (e.g., calcium carbonate, calcium sulfate, sodium hydrogencarbonate and sodium chloride) and plant powders (e.g., licorice powder and gentian powder); disintegrating agents such as starch, agar, gelatin powder, crystalline cellulose, carmellose sodium, carmellose calcium, calcium carbonate, sodium hydrogencarbonate and sodium alginate; lubricants such as magnesium stearate, talc, hydrogenated vegetable oil, macrogol and silicone oil; binders such as polyvinyl alcohol, hydroxypropyl cellulose, methyl cellulose, ethyl cellulose, carmellose, gelatin and starch paste; surfactants such as fatty acid esters; plasticizers such as glycerin, etc.

When the dosage form of the oral preparation is a liquid preparation such as syrup, the preparation can be prepared by adding water, sugars such as sucrose, sorbitol and fructose, glycols such as polyethylene glycol and propylene glycol, oils such as sesame oil, olive oil and soybean oil, antiseptics such as p-hydroxybenzoate esters, preservatives such as paraoxybenzoic acid derivatives (e.g., methyl paraoxybenzoate) and sodium benzoate, flavors such as strawberry flavor and peppermint, etc.

An example of the dosage form of the parenteral preparation is an injection solution. In the case of injection solution, it is possible to make the preparation using a solution which is isotonic to blood such as a carrier comprising a saline solution, a glucose solution or a mixture of a saline solution and a glucose solution.

The above-described antiseptics, preservatives, surfactants, etc. can also be added to the parenteral preparation.

When the muscle building agent and the preventive or therapeutic agent for muscle wasting of the present invention are administered to animals, preferably to mammals, the dose and administration schedule are as follows. When they are administered to a human being, the dose and administration schedule will vary depending upon the administration route, the age and body weight of a patient, and the nature or degree of severeness of the symptom to be treated. In the case of oral administration, the agents are usually administered in a dose of 0.1 mg to 50 g, preferably 1 mg to 10 g, more preferably 10 mg to 1 g in terms of CDP-choline per adult once to several times a day. In the case of parenteral administration such as intravenous administration, the agents are usually administered in a dose of 0.1 mg to 50 g, preferably 1 mg to 10 g, more preferably 10 mg to 1 g per adult once to several times a day. When the agents are administered to non-human animals, preferably to non-human mammals, the dose and administration schedule will vary depending upon the age and kind of the non-human animal, and the nature or degree of severeness of the symptom. The agents are usually administered in a dose of 0.1 mg to 10 g, preferably 1 mg to 5 g, more preferably 10 mg to 1 g per kg of body weight once to several times a day. In the case of parenteral administration such as intravenous administration, the agents are usually administered in a dose of 0.01 mg to 10 g, preferably 0.1 mg to 1 g per kg of body weight once to several times a day.

The food additive of the present invention can be prepared by the same methods as the above-described methods for preparing the oral preparations. The food additive is produced in the form, for example, of powder, granules, pellets, tablets or various liquid preparations usually by adding or dissolving other food additives according to need.

Other food additives include additives such as sweeteners, coloring agents, preservatives, thickening stabilizers, antioxidants, color developing agents, bleaching agents, antifungal agents, gum bases, bitter agents, enzymes, wax, sour agents, seasonings, emulsifiers, nutrient supplements, manufacture facilitating agents, flavors and spice extracts which are described in Food Additives Indication Pocket Book (Japan Food Additives Association, January 6, 1997). The carriers mentioned in the above description of tablets and the like may also be added.

Examples of the additives are given below.

Examples of the sweeteners are aspartame, licorice, stevia, xylose and rakanka (dried fruit of Momordica grosvenori*)*.

Examples of the coloring agents are carotenoid pigment, turmeric pigment, flavonoid, caramel pigment, oriental gromurell pigment, spirulina pigment, chlorophyll, *murasakiimo* (purple potato) pigment, *murasakiyamaimo* (purple yam) pigment, perilla pigment and blueberry pigment.

Examples of the preservatives are sodium sulfite, benzoic acid compounds, extract of Aralia cordata, extract of Styrax japonica, extract of Artemisia capillaris, sorbic acid compounds and propionic acid compounds.

Examples of the thickening stabilizers are gums such as gum arabic and xanthan gum, alginic acid compounds, chitin, chitosan, aloe extract, guar gum, hydroxypropyl cellulose, casein sodium, corn starch, carboxymethyl cellulose, gelatin, agar, dextrin, methyl cellulose, polyvinyl alcohol, microfibrous cellulose, microcrystalline cellulose, seaweed cellulose, sodium polyacrylate, sodium polyphosphate, carrageenan, cell wall of yeast, extract of konjac (Amorphophallus konjac), nata de coco and mannan.

Examples of the antioxidants are vitamin C compounds, sodium ethylenediaminetetraacetate, calcium ethylenediaminetetraacetate, erythorbic acid, oryzanol, catechin, quercetin, clove extract, enzyme-treated rutin, apple extract, sesame oil extract, dibutylhydroxytoluene, fennel extract, horseradish extract, water dropwort extract, tea extract, tempeh extract, extract of Houttuynia cordata, tocotrienol, tocopherols, rapeseed oil extract, green coffee extract, sunflower seed, ferulic acid, butylhydroxyanisole, blueberry leaf extract, propolis extract, hego-ginkgo leaf extract, hesperetin, pepper extract, garden balsam extract, gallic acid, bayberry extract, eucalyptus extract and rosemary extract.

An example of the color developing agent is sodium nitrite.

An example of the bleaching agent is sodium sulfite.

An example of the antifungal agent is orthophenylphenol.

Examples of the gum bases are methyl acetyl ricinoleate, Japanese lacquer wax, ester gum, elemi resin, urucury wax, ozocerite, opopanax resin, kauri gum, carnauba wax, guaiac resin, gutta katiau, gutta hangkang, guttapercha, glycerin fatty acid ester, spermaceti, copaiba balsam, copal resin, rubber, rice bran wax, sugar cane wax, shellac, jelutong, sucrose fatty acid ester, sorva, sorbitan fatty acid ester, talc, calcium carbonate, dammar resin, chicle, chilte, tunu, low-molecular rubber, paraffin wax, fir balsam, propylene glycol fatty acid ester, powdered pulp, powdered chaff, jojoba wax, polyisobutylene, polybutene, microcrystalline wax, mastic, massaranduba chocolate, beeswax and calcium phosphate.

Examples of the bitter agents are iso-alpha bitter acid, caffeine, extract of *kawaratake* (Coriolus versicolor), cinchona extract, Amur cork extract, gentian extract, spice extracts, enzyme-treated naringin, Jamaica quassia extract, theobromine, naringin, bitter ash extract, warmwood extract, isodonis extract, extract of himematsutake (Agaricus blazei), borapet, methylthioadenosine, litchi extract, olive tea, sour orange extract, hop extract and mugwort extract.

Examples of the enzymes are amylase, trypsin, rennet and lactic acid bacteria.

Examples of the wax are Japanese lacquer wax and vegetable wax.

Examples of the sour agents are adipic acid, itaconic acid, citric acid compounds, succinic acid compounds, sodium acetate, tartaric acid compounds, carbon dioxide, lactic acid, phytic acid, fumaric acid, malic acid and phosphoric acid.

Examples of the seasonings are amino acids such as asparagine, aspartic acid, glutamic acid, glutamine, alanine, isoleucine, glycine, serine, cystine, tyrosine, leucine and proline; nucleic acids such as sodium inosinate, sodium uridylate, sodium guanylate, sodium cytidylate, calcium ribonucleotide and sodium ribonucleotide; organic acids such as citric acid and succinic acid; potassium chloride; low-salt sodium solution from salt lake water; crude potassium chloride prepared from sea water; whey salt; tripotassium phosphate; dipotassium hydrogenphosphate; potassium dihydrogenphosphate; disodium hydrogenphosphate; sodium dihydrogenphosphate; trisodium phosphate; and chlorella extract.

Examples of the emulsifiers are fatty acid esters of glycerol, propylene glycol, sorbitan and sucrose, and lecithin.

Examples of the nutrient supplements are zinc salts, vitamin C compounds, various amino acids, 5-adenylic acid, iron chloride, hesperidin, various burnt calcium products, various unburnt calcium products, dibenzoylthiamine, calcium hydroxide, calcium carbonate, thiamine hydrochloride, dunaliella carotene, tocopherol, nicotinic acid, carrot carotene, palm oil carotene, calcium pantothenate, vitamin A, hydroxyproline, calcium dihydrogenpyrophosphate, ferrous pyrophosphate, ferric pyrophosphate, ferritin, heme iron, menaquinone, folic acid and riboflavin.

Examples of the manufacture facilitating agents are processing aids such as acetone and ion-exchange resin, extract of fig leaves, extract of ash of rice straw, kaolin, glycerin fatty acid ester, mulberry extract, bone ash, perilla extract, ginger extract, various tannins, paffia extract, extract of grape seeds, and ethanol.

An example of the flavor is vanilla essence.

An example of the spice extract is capsicum extract.

Each of the above-mentioned various kinds of additives may also be added to the muscle building agent and the preventive or therapeutic agent for muscle wasting of the present invention.

Examples of the food and drink of the present invention are foods and drinks to which CDP-choline or a salt thereof has been added.

The food and drink of the present invention can be processed and produced by general methods for the production of foods and drinks, modified only in that CDP-choline or a salt thereof is added to the food and drink.

The food and drink of the present invention may also be produced, for example, by using granulating methods such as fluidized bed granulation, stirring granulation, extrusion granulation, rolling granulation, air stream granulation, compression molding granulation, crushing granulation, spray granulation and jet granulation; coating methods such as pan coating, fluidized bed coating and dry coating; puffing methods such as puff drying, excess vapor method, foam-mat method and microwave-heating method; and extrusion methods using an extrusion granulator, an extruder, or the like.

Examples of the food and drink of the present invention include juice, soft drinks, tea, dairy products (e.g., lactic acid bacteria beverages, fermented milk, ice cream, butter, cheese, yogurt, processed milk and skim milk), meat products (e.g., ham, sausage and hamburger), fish meat paste products (e.g., *kamaboko, chikuwa* and *satsumaage*), egg products (e.g., *dashimaki* and *tamagodofu*), confectionery (e.g., cookies, jelly, chewing gum, candies and snacks), bread, noodles, pickles, smoked fish and meat, dried fish, *tsukudani* (food boiled down in soy), salted foods, soup and seasonings.

Further, the food and drink of the present invention may take forms such as a powder food, a sheet-shaped food, a bottled food, a canned food, a retort pouched food, a capsule food, a tablet food, a liquid food and a nutritional drink.

The food and drink of the present invention can be used as a health food, a functional food or the like for muscle building or for prevention or treatment of muscle wasting.

It is possible to add to the food and drink of the present invention the above-described food additives which can be added to the food and drink additive of the present invention, such as a sweetener, a coloring agent, a preservative, a thickening stabilizer, an antioxidant, a color developing agent, a bleaching agent, an antifungal agent, a gum base, a bitter agent, an enzyme, wax, a sour agent, a seasoning, an emulsifier, a nutrient supplement, a manufacture facilitating agent, a flavor and a spice extract.

The amount of CDP-choline or a salt thereof to be added to the food and drink of the present invention or the amount of the food additive of the present invention to be added to food and drink is appropriately selected depending upon the kind of the food and drink, the desired effect of ingestion of the food and drink, etc. so as to make the content of CDP-choline or a salt thereof usually 0.001 to 100 wt%, preferably 0.01 to 80 wt%, particularly preferably 0.1 to 60 wt%.

Although the oral dose, i.e. the ingestion amount of the food and drink of the present invention may vary depending upon the mode of ingestion, the age and body weight of the ingesting person, etc., it is usually 0.1 mg to 50 g, preferably 1 mg to 10 g, more preferably 10 mg to 1 g per adult per day in terms of CDP-choline or a salt thereof and it is ingested at once or in several portions daily. Although there is no particular limitation on the period of ingestion, it is usually from one day to five years, preferably from two weeks to one year.

The feed additive according to the present invention can be prepared by the same methods as in the case of the oral preparations of the present invention. The feed additive is produced in the form, for example, of powder, granules, pellets, tablets or various liquid preparations usually by adding or dissolving other feed additives according to need.

Examples of other feed additives include the above-described food additives which can be added to the food and drink additive of the present invention, such as a sweetener, a coloring agent, a preservative, a thickening stabilizer, an antioxidant, a color developing agent, a bleaching agent, an antifungal agent, a gum base, a bitter agent, an enzyme, wax, a sour agent, a seasoning, an emulsifier, a nutrient supplement, a manufacture facilitating agent, a flavor and a spice extract.

The feed of the present invention includes any feed for animals such as mammals, birds, reptiles, amphibians and fish used for building muscle or for prevention or treatment of muscle wasting. Examples of the feeds are feed for pets such as dogs, cats, rats and mice, feed for livestock such as cows, horses and pigs, feed for poultry such as hens and turkeys, and feed for cultivated fish such as sea breams and young yellowtails. The feed can be preferably used as feed for pets or poultry.

The feed of the present invention can be processed and produced by general methods for the production of feed, modified only in that CDP-choline or a salt thereof or the feed additive of the present invention is added to the feed.

Examples of the feeds include cereals, chaff and bran, vegetable oil cake, feed derived from animals, other feeds, purified products and a mixture thereof.

Examples of the cereals are milo, wheat, barley, oats, rye, unpolished rice, buckwheat, foxtail millet, common millet, Japanese millet, corn and soybean.

Examples of the chaff and bran are rice bran, defatted rice bran, wheat bran, wheat middlings, wheat germ, barley bran, pellet, corn bran and corn germ.

Examples of the vegetable oil cakes are soybean oil cake, soybean flour, linseed oil cake, cottonseed oil cake, peanut oil cake, safflower oil cake, coconut oil cake, palm oil cake, sesame oil cake, sunflower oil cake, rapeseed oil cake, kapok oil cake and mustard oil cake.

Examples of the feeds derived from animals are fish meal (e.g., northern ocean meal, imported meal, whole meal and coastal meal), fish soluble, meat meal, meat and bone meal, blood powder, decomposed hair, bone meal, by-product upon disposal of livestock, feather meal, silkworm pupa, skim milk powder, casein and dry whey.

Examples of other feeds are stalks and leaves of plants (e.g., alfalfa, hay cube, alfalfa leaf meal and powder of false acacia), by-products in corn-processing industry (e.g., corn gluten, meal, corn gluten feed and corn steep liquor), processed starch products (e.g., starch), fermentation industrial products (e.g., yeast, beer cake, malt root, alcohol cake and soy sauce cake), by-products in processing of agricultural products (e.g., processed citrus fruit cake, tofu (soybean curd) cake, coffee cake and cocoa cake) and others (e.g., cassava, broad bean, guar meal, seaweeds, krill, spirulina, chlorella and minerals).

Examples of the purified products are proteins (e.g., casein and albumin), amino acids, carbohydrates (e.g., starch, cellulose, sucrose and glucose), minerals and vitamins.

The feed of the present invention may also be produced, for example, by using granulating methods such as fluidized bed granulation, stirring granulation, extrusion granulation, rolling granulation, air stream granulation, compression molding granulation, crushing granulation, spray granulation and jet granulation; coating methods such as pan coating, fluidized bed coating and dry coating; puffing methods such as puff drying, excess vapor method, foam-mat method and microwave-heating method; and extrusion methods using an extrusion granulator, an extruder, or the like.

The amount of CDP-choline or a salt thereof or the feed additive to be added to the feed of the present invention is appropriately selected depending upon the kind of the feed, the desired effect of ingestion of the feed, etc. so as to make the content of CDP-choline or a salt thereof usually 0.001 to 100 wt%, preferably 0.01 to 80 wt%, particularly preferably 0.1 to 60 wt%.

Although the oral dose, i.e. the ingestion amount of the feed of the present invention may vary depending upon the mode of ingestion, the kind, age and body weight of the ingesting animal, etc., it is usually 0.1 mg to 10 g, preferably 1 mg to 5 g, more preferably 10 mg to 1 g per kg of body weight per day in terms of CDP-choline or a salt thereof and it is ingested at once or in several portions daily. Although there is no particular limitation on the period of ingestion, it is usually from one day to five years, preferably from two weeks to one year.

### Test Example 1

### Muscle-building Action of CDP-Choline

As test animals, 13 female 9-week-old rats of Fischer 344 strain (purchased from SLC Inc.) were used. The rats were preliminarily bred for 3 days in a chamber in which the temperature was adjusted to 22°C under a bright-dark cycle where 07:00 to 19:00 was the bright period, and access to water and feed was ad libitum. In the preliminary breeding, a feed prepared based on AIN-93M diet having the composition shown in Tables 1, 2 and 3 was given. After the preliminary breeding, the rats were divided into two groups, and a control feed was given to a control group consisting of 7 rats while a feed comprising CDP-choline (manufactured by Kyowa Hakko Kogyo Co., Ltd.) having the composition shown in Table 1 (hereinafter referred to as a CDP-choline feed) was given to the other group consisting of 6 rats for one day. Then, the right hind limb of each rat was immobilized in plaster cast under anesthesia with pentobarbital, followed by further feeding for one week under the above conditions. Thereafter, the rats were subjected to analysis with regard to the items shown below.

**Table 1:**

| Composition of Feed | | |
|---|---|---|
| Composition | Content (wt%) | |
| | Control feed (AIN-93M) | CDP-Choline feed |
| Corn starch | 46.5692 | 45.5692 |
| CDP-Choline | - | 1.00 |
| Milk casein | 14.00 | 14.00 |
| Pregelatinized corn starch | 15.50 | 15.50 |
| Granulated sugar | 10.00 | 10.00 |
| Refined soybean oil | 4.00 | 4.00 |
| Cellulose powder | 5.00 | 5.00 |
| Mineral mix | 3.50 | 3.50 |
| Vitamin mix | 1.00 | 1.00 |
| L-Cystine | 0.18 | 0.18 |
| Choline bitartrate | 0.25 | 0.25 |
| Tertiary-butylhydroquinone | 0.0008 | 0.0008 |
| Total | 100.00 | 100.00 |

**Table 2:**

| Composition of Mineral Mix | |
|---|---|
| Mineral Mix | |
| Composition | Content (wt%) |
| Calcium carbonate | 35.7 |
| Potassium dihydrogenphosphate | 25.0 |
| Potassium citrate monohydrate | 2.80 |
| Sodium chloride | 7.40 |
| Potassium sulfate | 4.66 |
| Magnesium oxide | 2.40 |
| Iron citrate monohydrate | 0.606 |
| Zinc oxide (5ZnO·2CO₂·H₂O) | 0.165 |
| Manganese carbonate | 0.063 |
| Copper carbonate [CuCO₃Cu(OH)₂·H₂O] | 0.030 |
| Potassium iodate | 0.001 |
| Sodium selenate | 0.001025 |
| Ammonium molybdate tetrahydrate | 0.000795 |
| Sodium silicate nonahydrate | 0.145 |
| Potassium chromium sulfate dodecahydrate | 0.02750 |
| Boric acid | 0.00815 |
| Sodium fluoride | 0.00635 |
| Nickel carbonate [NiCO₃·2Ni(OH₂)·4H₂O] | 0.00318 |
| Lithium chloride | 0.00174 |
| Ammonium vanadate | 0.00066 |
| Granulated sugar | 22.1026 |
| Total | 100.00 |

**Table 3:**

| Composition of Vitamin Mix | |
|---|---|
| Vitamin Mix | |
| Composition | Content (wt%) |
| Nicotinic acid | 0.30 |
| Ca DL-pantothenate | 0.32 |
| Vitamin B₆ | 0.07 |
| Vitamin B₁ | 0.06 |
| Vitamin B₂ | 0.06 |
| Folic acid | 0.02 |
| D-Biotin (2%) | 0.10 |
| Vitamin B₁₂ (0.1%) | 0.25 |
| Vitamin E (50%) | 1.50 |
| Vitamin A (500,000 IU/g) | 0.08 |
| Vitamin D₃ (500,000 IU/g) | 0.02 |
| Vitamin K₁ (Phylloquinone) | 0.01 |
| Granulated sugar | 97.21 |
| Total | 100.00 |

### (1) Body Weight

Measurement of body weight was conducted after a rat was anesthetized with pentobarbital and a plaster cast was removed. The result is shown in Table 4. In the table, n indicates the number of rats subjected to the test.

**Table 4:**

| Body Weight | | |
|---|---|---|
| Group | n | Body Weight (g) |
| Control | 7 | 143.7 ± 5.0 |
| CDP-Choline | 6 | 142.5 ± 4.3 |

Data are shown in terms of (mean value) ± (standard deviation).

As shown in Table 4, no difference in body weight due to the difference in ingested feed was observed.

### (2) Wet Weight of Muscle

In the test, two muscles, i.e., plantar muscle and soleus muscle located at the lower hind limbs of a rat, were used. Soleus muscle is a representative slow skeletal muscle in lower limbs where 80 to 90% of total muscle fibers are slow muscle fibers, and plantar muscle is a typical fast skeletal muscle where about 95% of total muscle fibers are fast muscle fibers.

The rat was killed after body weight measurement, and the soleus muscle and plantar muscle of both the right and left lower hind limbs were quickly excised and washed with a physiological saline solution. After extraneous connective tissues, tendons, nerves, etc. were removed, the muscle wet weights of soleus muscle and plantar muscle were measured. The relative wet weight of muscle immobilized in a cast was determined for each rat based on the wet weight of muscle which was not immobilized in a cast, taken as 100, and the decrease in muscle weight was expressed as the atrophy rate. The data on soleus muscle are shown in Table 5, and those on plantar muscle in Table 6.

**Table 5:**

| Wet Weight of Soleus Muscle | | | | |
|---|---|---|---|---|
| Group | n | Wet Weight of Soleus Muscle (mg) | | Atrophy Rate (%) |
| | | Immobilized | Non-immobilized | |
| Control | 7 | 63.6 ± 2.6 | 80.8 ± 6.3 | 20.9 ± 5.2 |
| CDP-Choline | 6 | 61.0 ± 5.2 | 77.2 ± 3.9 | 21.0 ± 6.5 |

Data are shown in terms of (mean value) ± (standard deviation).

**Table 6:**

| Wet Weight of Plantar Muscle | | | | |
|---|---|---|---|---|
| Group | n | Wet Weight of Plantar Muscle (mg) | | Atrophy Rate (%) |
| | | Immobilized | Non-immobilized | |
| Control | 7 | 100.6 ± 3.7 | 124.0 ± 7.3 | 18.7 ± 4.2 |
| CDP-Choline | 6 | 104.0 ± 2.7 | 123.8 ± 7.4 | 15.8 ± 5.8 |

Data are shown in terms of (mean value) ± (standard deviation).

As shown in Table 5, in soleus muscle, no difference in wet weight of muscle due to the ingested feed was observed in both the immobilized legs and non-immobilized legs. On the other hand, as shown in Table 6, in plantar muscle, the wet weight of the immobilized legs of the CDP-choline-administered group was significantly higher than the control group, and thus the atrophy rate was significantly lower.

### (3) Wet Weight of Muscle per Body Weight

The wet weight of muscle relative to body weight was calculated by measuring the wet weight of both right and left soleus muscles and plantar muscles and dividing the obtained value by body weight. The wet weight of muscle per body weight excludes the influence of the difference in body weight on each muscle wet weight and reflects the muscle amount per body weight. The wet weight of soleus muscle per body weight is shown in Table 7, and the wet weight of plantar muscle per body weight is shown in Table 8.

**Table 7:**

| Wet Weight of Soleus Muscle per Body Weight | | | |
|---|---|---|---|
| Group | n | Wet Weight of Soleus Muscle (mg/g body weight) | |
| | | Immobilized | Non-immobilized |
| Control | 7 | 0.443 ± 0.013 | 0.562 ± 0.041 |
| CDP-Choline | 6 | 0.428 ± 0.027 | 0.542 ± 0.030 |

Data are shown in terms of (mean value) ± (standard deviation).

**Table 8:**

| Wet Weight of Plantar Muscle per Body Weight | | | |
|---|---|---|---|
| Group | n | Wet Weight of Plantar Muscle (mg/g body weight) | |
| | | Immobilized | Non-immobilized |
| Control | 7 | 0.700 ± 0.017 | 0.862 ± 0.030 |
| CDP-Choline | 6 | 0.731 ± 0.029 | 0.870 ± 0.052 |

Data are shown in terms of (mean value) ± (standard deviation).

As shown in Table 7, in the wet weight of soleus muscle per body weight, no difference due to the ingested feed or the immobilization of leg was observed. On the other hand, as shown in Table 8, the plantar muscle weight per body weight in the case of the immobilized legs in the CDP-choline group was significantly higher than the control group.

The results shown in Table 6 and Table 8 revealed that CDP-choline increases the weight of plantar muscle, which is fast skeletal muscle.

### (4) Protein Content of Muscle

Soleus muscle was finely cut in a 1.15% potassium chloride solution and a homogenate thereof was prepared using a glass homogenizer. After the homogenate was appropriately diluted, its protein content was measured using the Protein Assay Kit of Biorad. A calibration curve was prepared using a 2 mg/ml BSA solution. The result of the measurement is shown in Table 9.

**Table 9:**

| Protein Content of Soleus Muscle | | | |
|---|---|---|---|
| Group | n | Protein Content (mg protein/g tissue) | |
| | | Immobilized | Non-immobilized |
| Control | 7 | 123.6 ± 23.4 | 161.4 ± 7.0 |
| CDP-Choline | 6 | 128.4 ± 10.0 | 173.2 ± 15.0 |
| | | | |

Data are shown in terms of (mean value) ± (standard deviation).

As shown in Table 9, the protein content of soleus muscle which had not been immobilized of the CDP-choline group was significantly higher than the control group.

### (5) Degradation Rate of Muscle Plasma Protein

Weighed soleus muscle was pre-incubated for 30 minutes in a Krebs-Henseleit bicarbonate buffer under aeration with 95% CO₂ - 5% O₂. The muscle was then transferred to fresh Krebs-Henseleit bicarbonate buffer, and incubated for 2 hours. The concentration of tyrosine in the buffer was measured, whereby the degradation rate of muscle plasma protein was calculated.

The concentration of tyrosine was measured by the fluorescence method of Waalkes, et al. using 1-nitroso-2-naphthol [J. Lab. Clin. Med., 50, 733 (1957)] (excitation wavelength: 435 nm; fluorescence wavelength: 545 nm). In order to prepare a calibration curve, a 5 nmol/ml tyrosine solution prepared as a standard solution was appropriately diluted and the relationship between its concentration and fluorescence intensity was plotted. By using the calibration curve thus prepared, the concentration of tyrosine in the buffer tested was determined from its fluorescence intensity. The result is shown in Table 10.

**Table 10:**

| Decomposition Rate of Muscle Plasma Protein of Soleus Muscle | | | |
|---|---|---|---|
| Group | n | Tyrosine (nmol/g tissue/2 hours) | |
| | | Immobilized | Non-immobilized |
| Control | 7 | 301.6 ± 80.0 | 170.8 ± 82.3 |
| CDP-Choline | 7 | 322.1 ± 98.0 | 135.5 ± 50.1 |

Data are shown in terms of (mean value) ± (standard deviation).

As shown in Table 10, in the group of rats with legs not immobilized in a cast and administered CDP-choline, the degradation rate of muscle plasma protein of soleus muscle was significantly lower than the control group.

The results shown in Tables 5, 7, 9 and 10 revealed that CDP-choline increases the protein content of soleus muscle and suppresses the degradation rate of soleus muscle plasma protein without changing the weight of soleus muscle.

### Test Example 2

### Action of CDP-Choline to Increase Intracellular Protein in vitro

### (1) Culturing of C2C12 Cells Derived from Mouse Myoblast Cells

C2C12 cells were purchased from Dainippon Pharmaceutical Co., Ltd. Culturing was carried out according to the method of Craig, et al. [Methods in Cell Biology, 52, 85 (1998)]. A medium for growth was prepared by addition of 50 ml of FBS (manufactured by Gibco; Cat. No. 10099-141) and 5 ml of penicillin-streptomycin (manufactured by Gibco; Cat. No. 15140-122) to 500 ml of D-MEM medium (manufactured by Gibco; Cat. No. 11885-084). A medium for differentiation was prepared by addition of 10 ml of inactivated horse serum (manufactured by Gibco; Cat. No. 26050-088) and 5 ml of penicillin-streptomycin to 500 ml of the D-MEM medium. C2C12 cells were seeded and cultured in a 75-cm² flask containing 15 ml of the medium for growth and were subcultured before reaching confluence. Culturing was carried out in a CO₂ incubator under 5% CO₂ concentration and 100% relative humidity.

The cell suspension diluted to 5 × 10⁴ cells/ml with the medium for growth was added to each well of type I collagen-coated 96-well plate (Iwaki; Cat. No. 4860-010) in an amount of 100 µl. After confirming that the growth was uniform, the medium was replaced by the differentiation medium. Myogenesis was confirmed under a microscope after culturing for 3 days, and then a CDP-choline-containing PBS (-) solution was added to the medium until its final concentration reached 1 mg/ml or 0.1 mg/ml, followed by further culturing. In the control group, PBS (-) solution was added instead of the CDP-choline-containing PBS (-) solution. Measurement of intracellular protein was conducted after culturing for 3 days.

### (2) Measurement of Amount of Intracellular Protein

The amount of intracellular protein was measured according to the method of Oliver, et al. [Journal of Cell Science, 92, 513 (1989)]. After the completion of culturing, the medium was removed from the collagen-coated plate and the plate was washed with a 0.15 mol/l sodium chloride solution. To each well was added 100 µl of a formalin solution (0.15 mol/l sodium chloride solution containing 10% formalin). After being allowed to stand for more than 30 minutes, the formalin solution was removed and 100 µl of a 1% Methylene Blue solution [10 mmol/l borate buffer (pH 8.5) containing 1% Methylene Blue] was added thereto. After being allowed to stand at room temperature for 30 minutes, the plate was washed four times with a 10 mmol/l borate buffer and moisture was wiped off. To each well was added 100 µl of an ethanolic hydrochloric acid solution (a solution containing ethanol and 0.1 mol/l hydrochloric acid in the ratio of 1:1), followed by light shaking. After the solution was appropriately diluted, the absorption at 650 nm was measured using a microplate reader. To determine the rate of increase of intracellular protein, the value of the test group (group to which CDP-choline was added) after culturing for 3 days was calculated based on the value before addition of CDP-choline (d0) taken as 0 and the concentration of intracellular protein in the control group (group to which no CDP-choline was added) after culturing for 3 days (d3) taken as 100. The result is shown in Table 11.

**Table 11:**

| Increase Rate of Intracellular Protein of C2C12 Cells | | |
|---|---|---|
| Group | CDP-Choline Concentration (mg/ml) | Increase Rate of Intracellular Protein |
| Control (d0) | 0 | 0 |
| Control (d3) | 0 | 100 |
| CDP-Choline | 0.1 | 134 |
| CDP-Choline | 1 | 141 |

Data are shown in terms of (mean value) ± (standard deviation).

As shown in Table 11, the amount of intracellular protein in C2C12 cells increased in a CDP-choline concentration dependent manner.

As the muscle weight of a leg immobilized in a plaster cast increased with the ingestion of CDP-choline, it has been confirmed that, in disuse muscular atrophy, CDP-choline has a muscle building action or a preventive or treating action on muscle wasting.

In the case of a non-immobilized leg, although no muscular atrophy as in the case of a leg immobilized in a cast was observed, restriction in daily life caused by immobilization of one of the legs lowers the quantity of motion of the other legs, whereby the other legs are likely to have lowered muscle quality. Accordingly, the increase in muscular protein amount in non-immobilized legs by the ingestion of CDP-choline means that a decrease in protein necessary for the maintenance of muscle is suppressed, and thus this confirms that CDP-choline has a preventive or therapeutic action upon muscle wasting caused by aging or lack of exercise.

Certain embodiments of the present invention are illustrated in the following examples. These examples are not to be construed as limiting the scope of the invention.

### Best Modes for Carrying Out the Invention

### Example 1

### Production of a Muscle Building Agent or a Preventive or Therapeutic Agent for Muscle Wasting Containing CDP-Choline

A muscle building agent or a preventive or therapeutic agent for muscle wasting was produced by mixing the following ingredients.

| | |
|---|---|
| CDP-Choline | 49 g |
| Pinedex #3 (Matsutani Chemical Industry Co., Ltd.) | 49 g |
| Ferric pyrophosphate | 0.1 g |
| Phoscal EFC (Nikko Fine Products Co., Ltd.) | 1 g |
| Vitamin mix (Merck & Co., Inc.) | 1 g |

### Example 2

### Production of a Muscle Building Agent or a Preventive or Therapeutic Agent for Muscle Wasting Containing CDP-Choline Hydrochloride

A muscle building agent or a preventive or therapeutic agent for muscle wasting was produced by mixing the following ingredients.

| | |
|---|---|
| CDP-Choline hydrochloride | 49 g |
| Pinedex #3 (Matsutani Chemical Industry Co., Ltd.) | 49 g |
| Ferric pyrophosphate | 0.1 g |
| Phoscal EFC (Nikko Fine Products Co., Ltd.) | 1 g |
| Vitamin mix (Merck & Co., Inc.) | 1 g |

### Example 3

### CDP-Choline-containing Feed for Muscle Building and for Prevention or Treatment of Muscle Wasting

Cookies (30 pieces) as dog feed for muscle building and for prevention or treatment of muscle wasting were produced by mixing the following ingredients.

| | |
|---|---|
| CDP-Choline | 0.5 g |
| Glucosamine | 0.5 g |
| Meat meal | 35.0 g |
| Corn starch | 40.0 g |
| Chicken extract | 5.0 g |
| Yeast extract | 5.0 g |
| Vegetable fat and oil | 5.0 g |
| Calcium lactate | 1.0 g |
| Sodium chloride | 1.0 g |
| Sodium hydrogenphosphate | 0.5 g |
| Magnesium carbonate | 0.5 g |
| Iron sulfate | 0.1 g |
| Vitamin B₁ | 0.0005 g |
| Vitamin B₂ | 0.0005 g |
| Vitamin E | 0.001 g |
| Niacin | 0.005 g |
| Vitamin A | 2000 IU |
| Vitamin D | 150 IU |
| Water | 6.3 g |

### Industrial Applicability

The present invention provides a muscle building agent, a preventive or therapeutic agent for muscle wasting, food and drink or feed for muscle building or for prevention or treatment of muscle wasting, and a food and drink additive or a feed additive for muscle building or for prevention or treatment of muscle wasting.

## Claims

1. A muscle building agent comprising cytidine diphosphate choline or a salt thereof.

2. The muscle building agent according to Claim 1, wherein the muscle is skeletal muscle.

3. A food and drink for building muscle comprising cytidine diphosphate choline or a salt thereof.

4. A food and drink for building muscle comprising cytidine diphosphate choline or a salt thereof added thereto.

5. The food and drink for building muscle according to Claim 3 or 4, wherein the muscle is skeletal muscle.

6. A feed for building muscle comprising cytidine diphosphate choline or a salt thereof.

7. A feed for building muscle comprising cytidine diphosphate choline or a salt thereof added thereto.

8. The feed for building muscle according to Claim 6 or 7, wherein the muscle is skeletal muscle.

9. A food additive for building muscle comprising cytidine diphosphate choline or a salt thereof.

10. A food additive for building muscle comprising cytidine diphosphate choline or a salt thereof added thereto.

11. The food additive for building muscle according to Claim 9 or 10, wherein the muscle is skeletal muscle.

12. A feed additive for building muscle comprising cytidine diphosphate choline or a salt thereof.

13. A feed additive for building muscle comprising cytidine diphosphate choline or a salt thereof added thereto.

14. The food additive for building muscle according to Claim 12 or 13, wherein the muscle is skeletal muscle.

15. A method for building muscle, which comprises administering to an animal an effective amount of cytidine diphosphate choline or a salt thereof.

16. The method according to Claim 15, wherein the animal is an animal other than a human being.

17. The method according to Claim 16, wherein the animal other than a human being is livestock, poultry or farm-raised fish.

18. A preventive or therapeutic agent for muscle wasting comprising cytidine diphosphate choline or a salt thereof.

19. The preventive or therapeutic agent for muscle wasting according to Claim 18, wherein the muscle is skeletal muscle.

20. A food and drink for the prevention or treatment of muscle wasting comprising cytidine diphosphate choline or a salt thereof.

21. A food and drink for the prevention or treatment of muscle wasting comprising cytidine diphosphate choline or a salt thereof added thereto.

22. The food and drink for the prevention or treatment of muscle wasting according to Claim 20 or 21, wherein the muscle is skeletal muscle.

23. A feed for the prevention or treatment of muscle wasting comprising cytidine diphosphate choline or a salt thereof.

24. A feed for the prevention or treatment of muscle wasting comprising cytidine diphosphate choline or a salt thereof added thereto.

25. The feed for the prevention or treatment of muscle wasting according to Claim 23 or 24, wherein the muscle is skeletal muscle.

26. A food additive for the prevention or treatment of muscle wasting comprising cytidine diphosphate choline or a salt thereof.

27. A food additive for the prevention or treatment of muscle wasting comprising cytidine diphosphate choline or a salt thereof added thereto.

28. The food additive for the prevention or treatment of muscle wasting according to Claim 26 or 27, wherein the muscle is skeletal muscle.

29. A feed additive for the prevention or treatment of muscle wasting comprising cytidine diphosphate choline or a salt thereof.

30. A feed additive for the prevention or treatment of muscle wasting comprising cytidine diphosphate choline or a salt thereof added thereto.

31. The food additive for the prevention or treatment of muscle wasting according to Claim 29 or 30, wherein the muscle is skeletal muscle.

32. A method for preventing or treating muscle wasting, which comprises administering to an animal an effective amount of cytidine diphosphate choline or a salt thereof.

33. The method according to Claim 32, wherein the animal is an animal other than a human being.

34. The method according to Claim 33, wherein the animal other than a human being is livestock, poultry or farm-raised fish.

35. Use of cytidine diphosphate choline or a salt thereof for the manufacture of a muscle building agent or food and drink, feed, food additive or feed additive for building muscle.

36. Use of cytidine diphosphate choline or a salt thereof for the manufacture of a preventive or therapeutic agent for muscle wasting or of food and drink, feed, food additive or feed additive for the prevention or treatment of muscle wasting.
